(19) Europäisches Patentamt
European Patent Office
Office européen des brevets

(11) Numéro de publication: **0 394 120 A1**

(12) **DEMANDE DE BREVET EUROPEEN**

(21) Numéro de dépôt: **90401036.0**

(22) Date de dépôt: **17.04.90**

(51) Int. Cl.[5]: **C07D 513/04, C07D 513/14, C07D 513/16, A61K 31/435, //(C07D513/04,275:00,221:00), (C07D513/14,275:00,221:00, 221:00),(C07D513/16,275:00, 221:00,221:00),(C07D513/16, 275:00,265:00,221:00)**

(30) Priorité: **18.04.89 FR 8905129**

(43) Date de publication de la demande:
**24.10.90 Bulletin 90/43**

(84) Etats contractants désignés:
**AT BE CH DE DK FR GB GR IT LI LU NL SE**

(71) Demandeur: **LABORATORIOS DEL DR. ESTEVE, S.A.**
**Av. Mare de Deu de Montserrat, 221**
**E-08026 Barcelona(ES)**

(72) Inventeur: **Pares Corominas, Juan**
**Padilla, 349, 30 3a**
**E-08025 Barcelona(ES)**
Inventeur: **Colombo Pinol, Augusto**
**Av. Chile, 36, 40 1a**
**E-08032 Barcelona(ES)**
Inventeur: **Frigola Constansa, Jordi**
**Av. Diagonal, 299 at.1a**
**E-08013 Barcelona(ES)**

(74) Mandataire: **Ahner, Francis et al**
**CABINET REGIMBEAU, 26, avenue Kléber**
**F-75116 Paris(FR)**

(54) **Dérivés d'isothiazolo-pyrydone azétidinyl substitués, leur préparation et leur application en tant que médicaments.**

(57) La présente invention se rapporte à des dérivés d'isothiazolo-pyrydone azétidinyl substitués, caractérisés en ce qu'ils répondent à la formule générale I :

ou sa formule tautomérique

**EP 0 394 120 A1**

Elle concerne également leur procédé de préparation et leur application en tant que médicaments à activité antimicrobienne.

# DERIVES D'ISOTHIAZOLO-PYRYDONE AZETIDINYL SUBSTITUES, LEUR PREPARATION ET LEUR APPLICATION EN TANT QUE MEDICAMENTS.

La présente invention se rapporte à des nouveaux dérivés azétidiniques d'isothiazolo-pyridone: 2,3,4,9-tétrahydroisothiazolo [5,4-b] quinoline-3,4-dione,2,3,4,9-tétrahydroisothiazolo [5,4-b] [1,8]-naphtyridine-3,4-dione, et 1,2,8,9-tétrahydro-7H-isothiazolo [4′,5′:5,6] pyrido [1,2,3-d e] benzoxazine-7,8-dione, les sels thérapeutiquement acceptables de ces composés, leur procédé de préparation, ainsi que leur application en tant que médicaments.

Les composés objet de la présente invention peuvent être utilisés dans l'industrie pharmaceutique comme intermédiaires et pour la préparation de médicaments.

On connaissait dejà des isothiazolo-naphtyridine et isothiazolo-quinoline, comme dans le brevet Eur. Pat. Appl. EP 227 088, et aussi des isothiazolo-pyrido-benzoxazines, comme dans le brevet Eur. Pat. Appl. EP 228661 mais en aucun de ces cas on a trouvé des examples avec des azétidines comme substituants.

Nous avons maintenant découvert que les nouveaux dérivés azétidiniques d'isothiazolo-naphtyridine, isothiazolo-quinoline, et isothiazolo-pyrido-benzoxazines, qui font l'objet de la présente invention, présentent une très bonne activité antimicrobienne.

Les composés objet de la présente invention répondent à la formule générale I

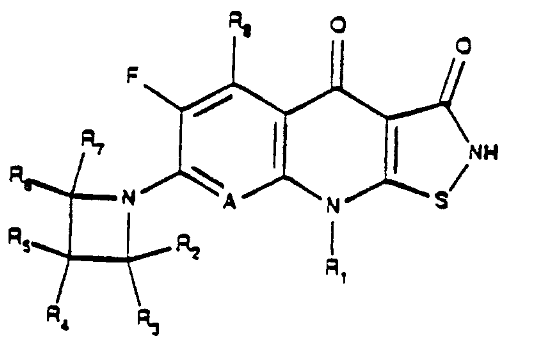

I

qu'on peut écrire aussi dans sa formule tautomérique II,

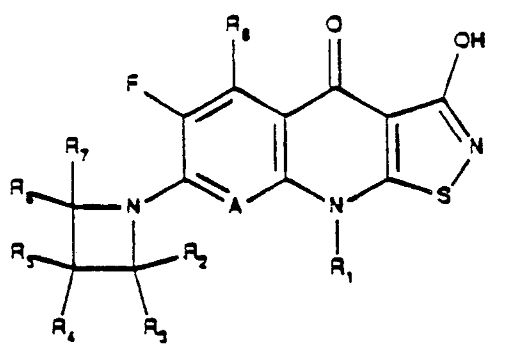

II

dans lesquelles A représente un atome d'azote ou bien un atome de carbone avec un atome d'hydrogène attaché (C-H), ou bien un atome de carbone avec un halogène attaché (C-X), dans ce cas X représente un atome de brome, de chlore ou de fluor.

$R_1$ représente un radical alkyle ou cycloalkyle inférieur, un radical halogénoalkyle inférieur, un radical aryle ou un radical aryle substitué, notamment par un ou plusieurs atome(s) de fluor.

$R_2$ et $R_7$, égaux ou différents, représentent un atome d'hydrogène ou un radical alkyle inférieur.

$R_3$, $R_5$ et $R_6$, égaux ou différents, représentent un atome d'hydrogène, un radical alkyle inférieur, un radical aminoalkyle, un radical alkylamino, un radical alkylaminoalkyle.

$R_4$, représente, un atome d'hydrogène, un radical alkyle inférieur, un radical hydroxyle, un radical amino, un radical aminoalkyle, un radical alkylamino, un radical dialkylamino, un radical hétérocyclique azoté pouvant être un cycle de trois à six maillons, un radical alkylaminoalkyle, un radical alkylcarboxamido, et dans ce

dernier cas, le radical alkyl pouvant être substitué par un ou plusieurs halogènes, un radical arylsulfonyloxy, un radical alkylsulfonyloxy, un radical carboxamido, pouvant être substitué ou non sur l'azote, ou un radical cyano.

$R_8$, représente un atome d'hydrogène, un radical nitro, un radical amino ou amino substitué.

A et $R_1$, peuvent former ensemble une liaison réprésentée par un groupe $C-CH_2-CH_2-CHR_9-$ ou $C-O-CH_2-CHR_9-$ dans lesquels $R_9$ représente un atome d'hydrogène ou un radical al kyle inférieur, et dans ce dernier cas on a un centre chiral avec une configuration "R" ou "S".

Les substituants azétidiniques peuvent avoir, selon le nombre, la nature et la position relative des substituants, jusqu'à trois centres chiraux, chacun d'eux avec une configuration "R" ou "S".

La stéréochimie des produits objet de la présente invention est déterminée par celle des produits de départ. Par sélection de la stéréoisomérie de chacun des produits de départ on peut obtenir tous les stéréoisomères possibles et dans le cas où le produit de réaction est un mélange stéréoisomérique, les composants peuvent être séparés et leur configuration établie par des procédés bien connus.

Les nouveaux dérivés de formule générale I peuvent être préparés, conformément à l'invention, selon la méthode suivante:

Par réaction d'un composé de formule générale III

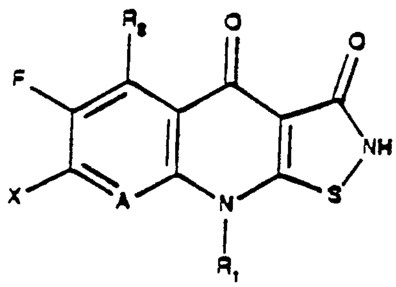

III

dans laquelle A, $R_1$ et $R_8$ ont les significations mentionées précédemment et X représente un atome d'halogène, de preférence un chlore ou un fluor, avec une azétidine, de formule générale IV

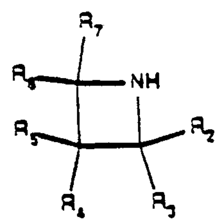

IV

dans laquelle $R_2$, $R_3$, $R_4$, $R_5$, $R_6$ et $R_7$ ont les significations mentionnées précédemment.

La réaction s'effectue en présence d'un solvant adéquat, par exemple le diméthylsulfoxyde, la diméthylformamide, la pyridine, les trialkylamines comme la triéthylamine, le chlorure de méthylène, le chloroforme, l'acétonitrile ou bien des éthers comme le tétrahydrofuranne ou le dioxanne, ou des mélanges de ces solvants.

Les températures les plus adéquates oscillent entre la température ambiante et la température de reflux du solvant, et le temps réactionnel est compris entre 1 heure et 24 heures.

Les composés hetérocycliques de formule générale III que l'on peut utiliser comme matières de départ pour préparer les composés de l'invention, sont des composés décrits, comme dans D.T.W. Chu, P.B. Fernandes, A.K. Claiborne, L. Shen et A.G. Pernet, Drugs Exptl. Chim. Res. 14(6), 379, (1988).

D'autre part, les composés de formule générale IV qui constituent les autres matières de départ pour la préparation des composés de l'invention selon la formule générale I, sont connus ou bien sont synthétisés comme par exemple dans A.G. Anderson et R. Lok, J. Org. Chem. 1972, 37, 3953 ou bien dans R.H. Higgins et N.H. Cromwell, J. Heterocycl. Chem., 1971, 8, 1059 et aussi dans N.H. Cromwell et B. Phillips, Chem. Rews., 1979, 79, 331.

Les azétidines de formule générale IV peuvent avoir, selon le nombre, la nature et la position relative des substituants, jusqu'à trois centres chiraux, et on peut obtenir les différents stéréoisomères soit par synthèse assymétrique soit par différentes sortes de séparations, selon les procédés connus dans la chimie

organique.

Les composés de formule générale I et leurs sels physiologiquement acceptables tels que les sels d'acides minéraux tels que les chlorhydrates, ou d'acides organiques tels que les toluènesulfonates ou méthylsulfonates, sont de préférence administrés sous forme de compositions pharmaceutiques.

Dans les exemples suivants on indique la préparation de nouveaux dérivés selon l'invention. On décrira également quelques formes d'emploi.

Les examples ci-après, donnés à simple titre d'illustration ne doivent cependant, en aucune façon, limiter l'étendue de l'invention.

## Exemple 1

Préparation de 9-cyclopropyl-6-fluoro-7-(3-amino-1-azétidinyl)-2,3,4,9-tétrahydroisothiazolo [5,4-b] quinoline-3,4-dione.

On maintient à reflux pendant 2 heures un mélange de 140 mg (0,475 mmoles) de 9-cyclopropyl-6,7-difluoro-2,3,4,9-tétrahydroisothiazolo [5,4-b] quinoline-3,4-dione, 300 mg (2 mmoles) de dichlorhydrate de 3-aminoazétidine et 1 ml de triéthylamine dans 5 ml de pyridine et 5 ml de diméthylsulfoxide. On filtre, on lave avec de l'eau et de l'éthanol, on sèche à chaud et on obtient 102 mg de 9-cyclopropyl-6-fluoro-7-(3-amino-1-azétidinyl)-2,3,4,9-tétrahydroisothiazolo [5,4-b] quinoline-3,4-dione, de point de fusion 270° C (d).

Données spectroscopiques: 1H RMN,δ, [DMSO-d6-ATFA]:

8,42 (é,2H); 7,60 (d,1H,J = 13Hz); 6,79 (d,1H,J = 7Hz); 4.16 (m,4H); 3,42 (m,2H); 1,20 (m,4H).

IR (KBr): 1629, 1602, 1492 $cm^{-1}$

## Exemple 2

Préparation de 9-cyclopropyl-6-fluoro-7-(3-méthylamino-1-azétidinyl)-2,3,4,9-tétrahydroisothiazolo [5,4-b] quinoline-3,4-dione.

On mantient à reflux pendant 1,5 heures un mélange de 500 mg (1,7 mmoles) de 9-cyclopropyl-6,7-difluoro-2,3,4,9-tétrahydroisothiazolo [5,4-b] quinoline-3,4-dione, 750 mg (3,4 mmoles) de chlorhydrate de 3-(trifluoroacétamido-N-méthyl) azétidine et 1,2 ml de triéthylamine dans 10 ml de pyridine et 10 ml de diméthylsulfoxide. On filtre, on lave avec de l'eau et de l'éthanol, on sèche à chaud et on obtient 430 mg de 9-cyclopropyl-6-fluoro-7-(N-méthyl-3-trifluoroacétamido-1-azétinidyl)-2,3,4,9-tétrahydroisothiazolo [5,4-b] quinoline-3,4-dione, de point de fusion 284-290° C, qui est ensuite hidrolisé en le chauffant avec de la soude 10% pendant 2 heures. On filtre, on lave avec de l'eau et de l'éthanol, on sèche à chaud et on obtient 340 mg de 9-cyclopropyl-6-fluoro-7-(3-méthylamino-1-azétidinyl)-2,3,4,9-tétrahydroisothiazolo [5,4-b] quinoline-3,4-dione, de point de fusion 270° C (d).

Données spectroscopiques: $^1$H-RMN,δ, [DMSO-d6-ATFA]:

9,22 (é,2H); 7,71 (d,1H,J=13Hz); 6,81 (d,1H,J=6Hz); 4,35 (m,4H); 3,45 (m,1H); 2,64 (s,3H); 1,29 (m,4H).

IR (KBr): 1629, 1588, 1497, 1431 $cm^{-1}$

## Exemple 3

Préparation de 9-cyclopropyl-6-fluoro-7-(3-diméthyl-amino-1-azétidinyl)-2,3,4,9-tétrahydroisothiazolo [5,4-b]-quinoline-3,4-dione.

On maintient à reflux pendant 2 heures un mélange de 150 mg (0,51 mmoles) de 9-cyclopropyl-6,7-difluoro-2,3,4,9-tétrahydroisothiazolo [5,4-b] quinoline-3,4-dione, 250 mg (1,45 mmoles) de dichlorhydrate de 3-diméthylaminoazétidine et 1 ml de triéthylamine dans 5 ml de pyridine et 5 ml de diméthylsulfoxide. On évapore sous vide la pyridine et la triéthylamine et, après refroidissement on filtre, on lave avec de l'eau et de l'éthanol, on sèche à chaud et on obtient 126 mg de 9-cyclopropyl-6-fluoro-7-(3-diméthylamino-1-azétidinyl)-2,3,4,9-tétrahydroisothiazolo [5,4-b] quinoline-3,4-dione, de point de fusion 287° C (d).

Données spectroscopiques: 1H RMN,δ, [DMSO-d6-ATFA]:
7,70 (d,1H,J = 13Hz); 6,77 (d,1H,J = 7Hz); 4,34 (m,4H); 3,43 (m,2H); 2,83 (s,6H); 1,20 (m,4H).
IR (KBr): 1640, 1612, 1501 $cm^{-1}$

Exemple 5

Préparation de 9-cyclopropyl-6-fluoro-7-(*trans*-3-amino-2-méthyl-1-azétidinyl)-2,3,4,9-tétrahydroisothiazolo [5,4-*b*] quinoline-3,4-dione.

On maintient à reflux pendant 2 heures un mélange de 150 mg (0,51 mmoles) de 9-cyclopropyl-6,7-difluoro-2,3,4,9-tétrahydroisothiazolo [5,4-*b*] quinoline-3,4-dione, 300 mg (1,9 mmoles) de dichlorhydrate de *trans*-3-amino-2-méthylazétidine et 1 ml de triéthylamine dans 5 ml de pyridine et 5 ml de diméthylsulfoxide. On filtre, on lave avec de l'eau et de l'éthanol, on sèche à chaud et on obtient 105 mg de 9-cyclopropyl-6-fluoro-7-(*trans*-3-amino-2-méthyl-1-azétidinyl)-2,3,4,9-tétrahydroisothiazolo [5,4-*b*] quinoline-3,4-dione, de point de fusion 264-7° C.
Données spectroscopiques: 1H RNM,δ, [DMSO-d6-ATFA]:
8,34 (é,2H); 7,75 (d,1H,J = 13Hz); 6,89 (d,1H,J = 7Hz); 4,44 (m,2H); 3,88 (m,1H); 3,44 (m,2H); 1,52 (d,3H); 1,20 (m,4H).
IR (KBr): 1628, 1592, 1486 $cm^{-1}$

Exemple 9

Préparation de 9-cyclopropyl-6-fluoro-7-(3-amino-3-méthyl-1-azétidinyl)-2,3,4,9-tétrahydroisothiazolo [5,4-*b*] quinoline-3,4-dione.

On maintient à reflux pendant 2 heures un mélange de 90 mg (0,3 mmoles) de 9-cyclopropyl-6,7-difluoro-2,3,4,9-tétrahydroisothiazolo [5,4-*b*] quinoline-3,4-dione, 200 mg (1,25 mmoles) de dichlorhydrate de 3-amino-3-methyl-azétidine et 1 ml de triéthylamine dans 5 ml de pyridine et 5 ml de diméthylsulfoxide. On filtre, on lave avec de l'eau et de l'éthanol, on sèche à chaud et on obtient 70 mg de 9-cyclopropyl-6-fluoro-7-(3-amino-3-méthyl-1-azétidinyl)-2,3,4,9-tétrahydroisothiazolo [5,4-*b*] quinoline-3,4-dione, de point de fusion 297-302° C.
Données spectroscopiques: 1H RMN,δ, [DMSO-d6-ATFA]:
8,50 (é,2H); 7,72 (d,1H,J = 13Hz); 6,79 (d,1H,J = 7Hz); 4,21 (m,4H); 3,45 (m,1H); 1,64 (s,3H); 1,25 (m,4H).
IR (KBr): 1629, 1592, 1492 $cm^{-1}$

Préparation du méthylsulfonate de 9-cyclopropyl-6-fluoro-7-(3-amino-3-méthyl-1-azétidinyl)-2,3,4,9-tétrahydroisothiazolo [5,4-*b*] quinoline-3,4-dione.

On suspend 60 mg (0,17 mmoles) de 9-cyclopropyl-6-fluoro-7-(3-amino-3-méthyl-1-azétidinyl)-2,3,4,9-tétrahydroisothiazolo [5,4-*b*] quinoline-3,4-dione dans 10 ml de méthanol et on additionne un excès d'acide méthylsulfonique. On agite pendant 15 minutes, on filtre, on lave avec du méthanol, on sèche à chaud et on obtient 55 mg du sel indiqué ci-dessus, de point de fusion 223-227° C.
Données spectroscopiques: 1H RMN,δ, [DMSO-d6-ATFA]:
8,44 (é,2H); 7,62 (d,1H,J = 13Hz); 6,72 (d,1H,J = 7Hz); 4,16 (m,4H); 3,43 (m,1H); 2,50 (s,3H); 1,60 (s,3H); 1,20 (m,4H).
IR (KBr): 1638, 1617, 1498 $cm^{-1}$

Exemple 10

Préparation de 9-cyclopropyl-6-fluoro-7-(3-méthylamino-3-méthyl-1-azétidinyl)-2,3,4,9-tétrahydroisothiazolo [5,4-*b*] quinoline-3,4-dione.

Par une procédure tout à fait semblable à celle décrite dans l'exemple 2, on obtient le 9-cyclopropyl-6-fluoro-7-(N-méthyl-3-trifluoroacétamido-3-méthyl-1-azétidinyl)-2,3,4,9-tétrahydroisothiazolo [5,4-b] quinoline-3,4-dione, de point de fusion 286-290 °C, qui est hidrolisé en le chauffant avec de la soude 10% pendant 2 heures, on obtient le 9-cyclopropyl-6-fluoro-7-(3-méthylamino-3-méthyl-1-azétidinyl)-2,3,4,9-tétrahydroisothiazolo [5,4-b] quinoline-3,4-dione, de point de fusion >300 °C.

Données spectroscopiques: $^1$H-RMN,δ, [DMSO-d6-ATFA]:
9,39 (é,2H); 7,80 (d,1H,J=13Hz); 6,90 (d,1H,J=6Hz); 4,28 (m,4H); 3,46 (m,1H); 2,70 (s,3H); 1,69 (s,3H); 1,22 (m,4H).

IR (KBr): 1625, 1597, 1495, 1474, 1428, 1385 $cm^{-1}$

Exemple 22

Préparation de 9-cyclopropyl-6-fluoro-7-(3-ethylamino-1-azétidinyl)-2,3,4,9-tétrahydroisothiazolo [5,4-b] quinoline-3,4-dione.

Par une procédure tout à fait semblable à celle décrite dans l'exemple 2, on obtient le 9-cyclopropyl-6-fluoro-7-(N-ethyl-3-trifluoroacétamido-1-azétidinyl)-2,3,4,9-tétrahydroisothiazolo [5,4-b] quinoline-3,4-dione, de point de fusion 256-259 °C, qui est hidrolisé en le chauffant avec de la soude 10% pendant 2 heures, on obtient le 9-cyclopropyl-6-fluoro-7-(3-ethylamino-1-azétidinyl)-2,3,4,9-tétrahydroisothiazolo [5,4-b] quinoline-3,4-dione, de point de fusion 231-236 °C.

Données spectroscopiques: $^1$H-RNM,δ, [DMSO-d6-ATFA]:
9,39 (é,2H); 7,69 (d,1H,J=13Hz); 6,80 (d,1H,J=6Hz); 4,29 (m,4H); 3,43 (m,1H); 3,03 (m,2H); 1,31 (m,7H).

IR (KBr): 1628, 1601, 1492, 1472, 1422 $cm^{-1}$

Exemple 29

Préparation de 9-cyclopropyl-6,8-difluoro-7-(trans-3-amino-2-méthyl-1-azétidinyl)-2,3,4,9-tétrahydroisothiazolo [5,4-b] quinoline-3,4-dione.

On mantient à reflux pendant 2 heures un mélange de 125 mg (0,4 mmoles) de 9-cyclopropyl-6,7,8-trifluoro-2,3,4,9-tétrahydroisothiazolo [5,4-b] quinoline-3,4-dione, 128 mg (0,8 mmoles) de dichlorhydrate de trans-2-méthyl-3-aminoazétidine et 0,34 ml de triéthylamine dans 4 ml de pyridine et 4 ml de diméthylsulfoxide. On filtre, on lave avec de l'eau et de l'éthanol, on sèche à chaud et on obtient 93 mg de 9-cyclopropyl-6,8-difluoro-7-(trans-3-amino-2-méthyl-1-azétinidyl)-2,3,4,9-tétrahydroisothiazolo [5,4-b] quinoline-3,4-dione, de point de fusion 268-270 °C.

Données spectroscopiques: $^1$H-RNM,δ, [DMSO-d6-ATFA]:
8,35 (é,3H); 7,66 (d,1H,J=13Hz); 4,70 (m,2H); 4,10 (m,1H); 3,78 (m,2H); 1,53 (d,3H,J=6Hz); 1,15 (m,4H).

IR (KBr): 1623, 1608, 1605, 1465, 1424 $cm^{-1}$

Exemple 33

Préparation de 9-cyclopropyl-6,8-difluoro-8-(3-amino-3-méthyl-1-azétidinyl)-2,3,4,9-tétrahydroisothiazolo [5,4-b] quinoline-3,4-dione

On maintient à reflux pendant 2 heures un mélange de 250 mg (0,8 mmoles) de 9-cyclopropyl-6,7,8-trifluoro-2,3,4,9-tétrahydroisothiazolo [5,4-b] quinoline-3,4-dione, 255 mg (1,6 mmoles) de dichlorhydrate de 3-méthyl-3-aminoazétidine et 0,6 ml de triéthylamine dans 10 ml de pyridine, on évapore sous vide, on additionne un mélange d'éthanol avec de l'eau, on filtre et on lave. On obtient ainsi 0.225 g de 9-cyclopropyl-6,8-difluoro-7-(3-amino-3-méthyl-1-azétidinyl)-2,3,4,9-tétrahydroisothiazolo [5,4-b] quinoline-3,4-dione, de point de fusion 294-300 °C.

Données spectroscopiques:
IR (KBr): 1630, 1602, 1469 $cm^{-1}$

Préparation du méthylsulfonate de 9-cyclopropyl-6,8-difluoro-7-(3-amino-3-méthyl-1-azétidinyl)-2,3,4,9-tétrahydroisothiazolo [5,4-b] quinoline-3,4-dione.

On suspend 120 mg (0,32 mmoles) de 9-cyclopropyl-6,8-difluoro-7-(3-amino-3-méthyl-1-azétidinyl)-2,3,4,9-tétrahydroisothiazolo [5,4-b] quinoline-3,4-dione dans 10 ml de méthanol et on additionne un excès d'acide méthylsulfonique. On agite pendant 15 minutes, on filtre, on lave avec du méthanol, on sèche à chaud et on obtient 120 mg du sel indiqué ci-dessus, de point de fusion 248-253° C.

Données spectroscopiques: 1H RMN,δ, [DMSO-d6-ATFA]:

8,40 (é,2H); 7,62 (d,1H,J = 12Hz); 4,35 (m,4H); 3,70 (m,1H); 2,50 (s,3H); 1,55 (s,1H); 1,07 (m,4H).

IR (KBr): 1648, 1619, 1468 $cm^{-1}$

En utilisant le 9-cyclopropyl-6-fluoro-2,3,4,9-tétrahydroisothiazolo [5,4-b] quinoline-3,4-dione comme produit de départ on peut obtenir, par la même procédure des exemples 1, 3 et 5, les produits des exemples 1 à 24 (Tableau I); en utilisant le 9-cyclopropyl-6,7-difluoro-2,3,4,9-tétrahydroisothiazolo [5,4-b] [1,8] quinoline-3,4-dione comme produit de départ on peut obtenir, par la même procédure de l'exemple 33, les produits des exemples 25 à 48 (Tableau I); en utilisant le 9-cyclopropyl-6,7-difluoro-2,3,4,9-tétrahydroisothiazolo [5,4-b] [1,8] naphtyridine-3,4-dione comme produit de départ on peut obtenir, par la même procédure de l'exemple 5, les produits des exemples 49 à 72 (Tableau I); en utilisant comme produit de départ le 1-méthyl-4,5-difluoro-1,2,8,9 tétrahydro-7H-isothiazolo [4′,5′;5,6] pyrido [1,2,3-d e] [1,4] benzoxazine-7,8-dione on peut obtenir, par la même procédure de l'exemple 5, les produits des exemples 73 à 96.

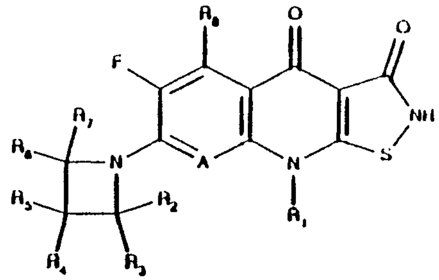

| Exemple | A | $R_1$ | $R_2$ | $R_3$ | $R_4$ | $R_5$ | $R_6$ | $R_7$ | $R_8$ | FORMULE |
|---|---|---|---|---|---|---|---|---|---|---|
| 1 | CH | | H | H | $H_2N$ | H | H | H | H | $C_{16}H_{15}FN_4O_2S$ |
| 2 | CH | | H | H | MeNH | H | H | H | H | $C_{17}H_{17}FN_4O_2S$ |
| 3 | CH | | H | H | $Me_2N$ | H | H | H | H | $C_{17}H_{19}FN_4O_2S$ |
| 4 | CH | | H | H | HO | H | H | H | H | $C_{16}H_{14}FN_3O_3S$ |
| 5 | CH | | H | H | $H_2N$ | H | Me | H | H | $C_{17}H_{17}FN_4O_2S$ |
| 6 | CH | | H | H | MeNH | H | Me | H | H | $C_{18}H_{19}FN_4O_2S$ |
| 7 | CH | | H | H | $Me_2N$ | H | Me | H | H | $C_{19}H_{21}FN_4O_2S$ |
| 8 | CH | | H | H | HO | H | Me | H | H | $C_{17}H_{16}FN_3O_3S$ |
| 9 | CH | | H | H | $H_2N$ | Me | H | H | H | $C_{17}H_{17}FN_4O_2S$ |
| 10 | CH | | H | H | MeNH | Me | H | H | H | $C_{18}H_{19}FN_4O_2S$ |
| 11 | CH | | H | H | $Me_2N$ | Me | H | H | H | $C_{19}H_{21}FN_4O_2S$ |
| 12 | CH | | H | H | HO | Me | H | H | H | $C_{17}H_{16}FN_3O_3S$ |
| 13 | CH | | H | Me | $H_2N$ | H | Me | H | H | $C_{18}H_{19}FN_4O_2S$ |

TABLEAU 1 (Cont.)

| Exemple | A | $R_1$ | $R_2$ | $R_3$ | $R_4$ | $R_5$ | $R_6$ | $R_7$ | $R_8$ | FORMULE |
|---|---|---|---|---|---|---|---|---|---|---|
| 14 | CH | 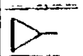 | H | Me | MeNH | H | Me | H | H | $C_{19}H_{21}FN_4O_2S$ |
| 15 | CH | 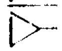 | H | Me | $Me_2N$ | H | Me | H | H | $C_{20}H_{23}FN_4O_2S$ |
| 16 | CH | 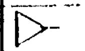 | H | Me | HO | H | Me | H | H | $C_{18}H_{18}FN_3O_3S$ |
| 17 | CH | 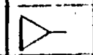 | H | Me | $H_2N$ | Me | H | H | H | $C_{18}H_{19}FN_4O_2S$ |
| 18 | CH | 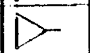 | H | Me | MeNH | Me | H | H | H | $C_{20}H_{23}FN_4O_2S$ |
| 19 | CH | 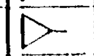 | H | Me | $Me_2N$ | Me | H | H | H | $C_{20}H_{23}FN_4O_2S$ |
| 20 | CH | 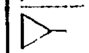 | H | Me | HO | Me | H | H | H | $C_{18}H_{18}FN_3O_3S$ |
| 21 | CH | 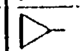 | H | H | $H_2NCH_2$ | H | H | H | H | $C_{17}H_{17}FN_4O_2S$ |
| 22 | CH | 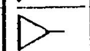 | H | H | EtNH | H | H | H | H | $C_{18}H_{20}FN_4O_2S$ |
| 23 | CH | 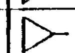 | H | H | $H_2NCH_2$ | Me | H | H | H | $C_{18}H_{19}FN_4O_2S$ |
| 24 | CH | 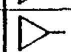 | H | H | $H_2NCH_2$ | H | Me | H | H | $C_{18}H_{19}FN_4O_2S$ |
| 25 | CF | 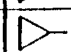 | H | H | $H_2N$ | H | H | H | H | $C_{16}H_{16}F_2N_4O_2S$ |
| 26 | CF | 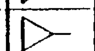 | H | H | MeNH | H | H | H | H | $C_{17}H_{18}F_2N_4O_2S$ |
| 27 | CF | 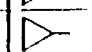 | H | H | $Me_2N$ | H | H | H | H | $C_{18}H_{20}F_2N_4O_2S$ |
| 28 | CF | 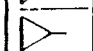 | H | H | HO | H | H | H | H | $C_{16}H_{15}F_2N_3O_3S$ |
| 29 | CF | 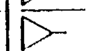 | H | H | $H_2N$ | H | Me | H | H | $C_{17}H_{18}F_2N_4O_2S$ |
| 30 | CF | 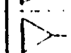 | H | H | MeNH | H | Me | H | H | $C_{18}H_{20}F_2N_4O_2S$ |
| 31 | CF | 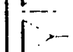 | H | H | $Me_2N$ | H | Me | H | H | $C_{19}H_{22}F_2N_4O_2S$ |
| 32 | CF | 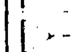 | H | H | HO | H | Me | H | H | $C_{17}H_{17}F_2N_3O_3S$ |
| 33 | CF | 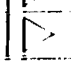 | H | H | $H_2N$ | Me | H | H | H | $C_{17}H_{18}F_2N_4O_2S$ |

TABLEAU I (Cont.)

| Exemple | A | $R_1$ | $R_2$ | $R_3$ | $R_4$ | $R_5$ | $R_6$ | $R_7$ | $R_8$ | FORMULE |
|---|---|---|---|---|---|---|---|---|---|---|
| 34 | CF | 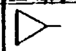 | H | H | MeNH | Me | H | H | H | $C_{18}H_{18}F_2N_4O_2S$ |
| 35 | CF | 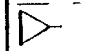 | H | H | $Me_2N$ | Me | H | H | H | $C_{19}H_{20}F_2N_4O_2S$ |
| 36 | CF | 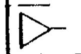 | H | H | HO | Me | H | H | H | $C_{17}H_{15}F_2N_3O_3S$ |
| 37 | CF | 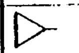 | H | Me | $H_2N$ | H | Me | H | H | $C_{18}H_{18}F_2N_4O_2S$ |
| 38 | CF | 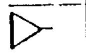 | H | Me | MeNH | H | Me | H | H | $C_{19}H_{20}F_2N_4O_2S$ |
| 39 | CF | 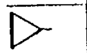 | H | Me | $Me_2N$ | H | Me | H | H | $C_{20}H_{22}F_2N_4O_2S$ |
| 40 | CF | 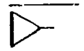 | H | Me | HO | H | Me | H | H | $C_{18}H_{17}F_2N_3O_3S$ |
| 41 | CF | 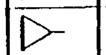 | H | Me | $H_2N$ | Me | H | H | H | $C_{18}H_{18}F_2N_4O_2S$ |
| 42 | CF | 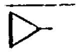 | H | Me | MeNH | Me | H | H | H | $C_{19}H_{20}F_2N_4O_2S$ |
| 43 | CF | 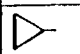 | H | Me | $Me_2N$ | Me | H | H | H | $C_{20}H_{22}F_2N_4O_2S$ |
| 44 | CF | 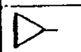 | H | Me | HO | Me | H | H | H | $C_{18}H_{17}F_2N_3O_3S$ |
| 45 | CF | 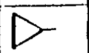 | H | H | $H_2NCH_2$ | H | H | H | H | $C_{17}H_{16}F_2N_4O_2S$ |
| 46 | CF | 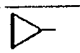 | H | H | $EtNHCH_2$ | H | H | H | H | $C_{19}H_{21}F_2N_4O_2S$ |
| 47 | CF | 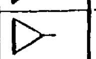 | H | H | $H_2NCH_2$ | Me | H | H | H | $C_{18}H_{18}F_2N_4O_2S$ |
| 48 | CF | 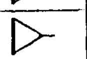 | H | H | $H_2NCH_2$ | H | Me | H | H | $C_{18}H_{18}F_2N_4O_2S$ |
| 49 | N | 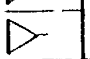 | H | H | $H_2N$ | H | H | H | H | $C_{15}H_{14}FN_5O_2S$ |
| 50 | N | 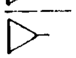 | H | H | MeNH | H | H | H | H | $C_{16}H_{16}FN_5O_2S$ |
| 51 | N | 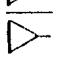 | H | H | $Me_2N$ | H | H | H | H | $C_{17}H_{18}FN_5O_2S$ |
| 52 | N | 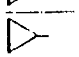 | H | H | HO | H | H | H | H | $C_{15}H_{13}FN_4O_3S$ |
| 53 | N | 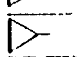 | H | H | $H_2N$ | H | Me | H | H | $C_{16}H_{16}FN_5O_2S$ |

TABLEAU I (Cont.)

| Exemple | A | $R_1$ | $R_2$ | $R_3$ | $R_4$ | $R_5$ | $R_6$ | $R_7$ | $R_8$ | FORMULE |
|---|---|---|---|---|---|---|---|---|---|---|
| 54 | N | 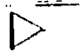 | H | H | MeNH | H | Me | H | H | $C_{17}H_{18}FN_5O_2S$ |
| 55 | N | 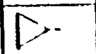 | H | H | $Me_2N$ | H | Me | H | H | $C_{18}H_{20}FN_5O_2S$ |
| 56 | N | 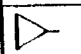 | H | H | HO | H | Me | H | H | $C_{16}H_{15}FN_4O_3S$ |
| 57 | N | 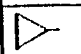 | H | H | $H_2N$ | Me | H | H | H | $C_{16}H_{16}FN_5O_2S$ |
| 58 | N | 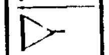 | H | H | MeNH | Me | H | H | H | $C_{17}H_{18}FN_5O_2S$ |
| 59 | N | 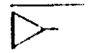 | H | H | $Me_2N$ | Me | H | H | H | $C_{18}H_{20}FN_5O_2S$ |
| 60 | N | 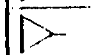 | H | H | HO | Me | H | H | H | $C_{16}H_{15}FN_4O_3S$ |
| 61 | N | 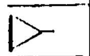 | H | Me | $H_2N$ | H | Me | H | H | $C_{17}H_{18}FN_5O_2S$ |
| 62 | N | 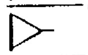 | H | Me | MeNH | H | Me | H | H | $C_{18}H_{20}FN_5O_2S$ |
| 63 | N | 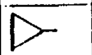 | H | Me | $Me_2N$ | H | Me | H | H | $C_{19}H_{22}FN_5O_2S$ |
| 64 | N | 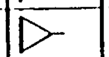 | H | Me | HO | H | Me | H | H | $C_{17}H_{17}FN_4O_3S$ |
| 65 | N | 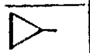 | H | Me | $H_2N$ | Me | H | H | H | $C_{17}H_{18}FN_5O_2S$ |
| 66 | N | 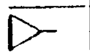 | H | Me | MeNH | Me | H | H | H | $C_{18}H_{20}FN_5O_2S$ |
| 67 | N | 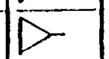 | H | Me | $Me_2N$ | Me | H | H | H | $C_{19}H_{22}FN_5O_2S$ |
| 68 | N | 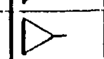 | H | Me | HO | Me | H | H | H | $C_{17}H_{17}FN_4O_3S$ |
| 69 | N | 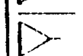 | H | H | $H_2NCH_2$ | H | H | H | H | $C_{16}H_{16}FN_5O_2S$ |
| 70 | N | 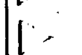 | H | H | $EtNHCH_2$ | H | H | H | H | $C_{18}H_{20}FN_5O_2S$ |
| 71 | N | 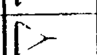 | H | H | $H_2NCH_2$ | Me | H | H | H | $C_{17}H_{18}FN_5O_2S$ |
| 72 | N | 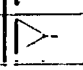 | H | H | $H_2NCH_2$ | H | Me | H | H | $C_{17}H_{18}FN_5O_2S$ |

TABLEAU I (Cont.)

| Exemple | A - $R_1$ | $R_2$ | $R_3$ | $R_4$ | $R_5$ | $R_6$ | $R_7$ | $R_8$ | FORMULE |
|---|---|---|---|---|---|---|---|---|---|
| 73 | $C-O-CH_2-CH(CH_3)-$ | H | H | $H_2N$ | H | H | H | H | $C_{16}H_{15}FN_4O_3S$ |
| 74 | $C-O-CH_2-CH(CH_3)-$ | H | H | MeNH | H | H | H | H | $C_{17}H_{17}FN_4O_3S$ |
| 75 | $C-O-CH_2-CH(CH_3)-$ | H | H | $Me_2N$ | H | H | H | H | $C_{18}H_{19}FN_4O_3S$ |
| 76 | $C-O-CH_2-CH(CH_3)-$ | H | H | HO | H | H | H | H | $C_{16}H_{14}FN_3O_4S$ |
| 77 | $C-O-CH_2-CH(CH_3)-$ | H | H | $H_2N$ | H | Me | H | H | $C_{17}H_{17}FN_4O_3S$ |
| 78 | $C-O-CH_2-CH(CH_3)-$ | H | H | MeNH | H | Me | H | H | $C_{18}H_{19}FN_4O_3S$ |
| 79 | $C-O-CH_2-CH(CH_3)-$ | H | H | $Me_2N$ | H | Me | H | H | $C_{19}H_{21}FN_4O_3S$ |
| 80 | $C-O-CH_2-CH(CH_3)-$ | H | H | HO | H | Me | H | H | $C_{17}H_{16}FN_3O_4S$ |
| 81 | $C-O-CH_2-CH(CH_3)-$ | H | H | $H_2N$ | Me | H | H | H | $C_{17}H_{17}FN_4O_3S$ |
| 82 | $C-O-CH_2-CH(CH_3)-$ | H | H | MeNH | Me | H | H | H | $C_{18}H_{19}FN_4O_3S$ |
| 83 | $C-O-CH_2-CH(CH_3)-$ | H | H | $Me_2N$ | Me | H | H | H | $C_{19}H_{21}FN_4O_3S$ |
| 84 | $C-O-CH_2-CH(CH_3)-$ | H | H | HO | Me | H | H | H | $C_{17}H_{16}FN_3O_4S$ |
| 85 | $C-O-CH_2-CH(CH_3)-$ | H | Me | $H_2N$ | H | Me | H | H | $C_{18}H_{19}FN_4O_3S$ |
| 86 | $C-O-CH_2-CH(CH_3)-$ | H | Me | MeNH | H | Me | H | H | $C_{19}H_{21}FN_4O_3S$ |
| 87 | $C-O-CH_2-CH(CH_3)-$ | H | Me | $Me_2N$ | H | Me | H | H | $C_{20}H_{23}FN_4O_3S$ |
| 88 | $C-O-CH_2-CH(CH_3)-$ | H | Me | HO | H | Me | H | H | $C_{18}H_{18}FN_3O_4S$ |
| 89 | $C-O-CH_2-CH(CH_3)-$ | H | Me | $H_2N$ | Me | H | H | H | $C_{18}H_{19}FN_4O_3S$ |
| 90 | $C-O-CH_2-CH(CH_3)-$ | H | Me | MeNH | Me | H | H | H | $C_{19}H_{21}FN_4O_3S$ |

TABLEAU I (Cont.)

| Exemple | A - $R_1$ | $R_2$ | $R_3$ | $R_4$ | $R_5$ | $R_6$ | $R_7$ | $R_8$ | FORMULE |
|---|---|---|---|---|---|---|---|---|---|
| 91 | $C-O-CH_2-CH(CH_3)-$ | H | Me | $Me_2N$ | Me | H | H | H | $C_{20}H_{23}FN_4O_3S$ |
| 92 | $C-O-CH_2-CH(CH_3)-$ | H | Me | HO | Me | H | H | H | $C_{18}H_{18}FN_3O_4S$ |
| 93 | $C-O-CH_2-CH(CH_3)-$ | H | H | $H_2NCH_2$ | H | H | H | H | $C_{17}H_{17}FN_4O_3S$ |
| 94 | $C-O-CH_2-CH(CH_3)-$ | H | H | $EtNHCH_2$ | H | H | H | H | $C_{19}H_{22}FN_4O_3S$ |
| 95 | $C-O-CH_2-CH(CH_3)-$ | H | H | $H_2NCH_2$ | Me | H | H | H | $C_{18}H_{19}FN_4O_3S$ |
| 96 | $C-O-CH_2-CH(CH_3)-$ | H | H | $H_2NCH_2$ | H | Me | H | H | $C_{18}H_{19}FN_4O_3S$ |

## Activité biologique

L'activité pharmacologique antimicrobienne de ces composés a été étudiée selon les réferences exposées ci-dessous.

Activité pharmacologique antimicrobienne (G. L. Daquet et Y. A. Chabbect, techniques en bactériologie, Vol 3, Flammarion Médecine-Sciences, Paris 1972, et W. B. Hugo et A. D. Rusell, Pharmaceutical Microbiology, Blackwell Scientific Publications, London, 1977.

Milieu de culture et solvant:

Agar d'antibiotiques nº 1 (Oxoid CM 327)

Bouillon de triptone-soja (Oxoid CM 129)

Solution Physiologique Ringer 1/4 (Oxoid BR 52)

Agar Dextrose (BBL 11165)

Microorganismes:

*"Bacillus subtilis" ATCC 6633*

*"Citrobacter freundii" ATCC 112606*

*"Enterobacter aerogenes" ATCC 15038*

*"Enterobacter cloacae" ATCC 23355*

*"Bacillus cereus" ATCC 1178*

*"Escherichia coli" ATCC 10799*

*"Escherichia coli" ATCC 23559*

*"Klebsiella pneumoniae" ATCC 10031*

*"Proteus vulgaris" ATCC 8427*

*"Morg. morganii" ATCC 8019*

*"Pseudomonas aeruginosa" ATCC 9721*

*"Pseudomonas aeruginosa" ATCC 10145*

*"Salmonella typhimurium" ATCC 14028*

*"Salmonella typhimurium" ATCC 6539*

*"Serratia marcescens" ATCC 13880*

*"Shigella flexnerii" ATCC 12022*

*"Staphylococcus epidermis ATCC 155-1*

*"Stapylococcus aureus ATCC 25178*

*"Streptococcus faecalis" ATCC 10541*

Préparation des inoculations.

Chacun des microorganismes est ensemencé par strie dans des tubes d'Agar d'antibiotiques nº1, et mis en incubation pendant 20 heures à 37° C. Ensuite on prend une anse de culture, on ensemence dans un bouillon de Triptone-soja et on incube pendant 20 heures à 37° C. On dilue à 1/4 la culture obtenue avec une solution physiologique Ringer, de façon à obtenir une suspension normalisée de $10^{-10}$ ufc/ml pour chaque organisme.

Préparation du milieu contenant les dérivés de formule générale I:

A partir d'une solution de 100 µg/ml, chaque produit est dilué dans l'Agar Dextrose (préalablement fondu et maintenu à 50° C) par dilutions successives de façon à obtenir les concentrations suivantes: 64 - 32 - 16 - 8 - 4-2 - 1 - 0,5 - 0,25 - 0,125 µg de dérivé/ml du milieu.

Postérieurement, chaque concentration de chaque produit est répartie dans des boîtes de Petri de 10 cm de diamètre, à raison de 10 ml de milieu par boîte et autant de boîtes que de microorganismes à tester.

Une fois le milieu refroidi, les boîtes sont ensemencées avec les inoculations à raison de 0,4 ml d'inoculation par boîte. On les étend avec une anse de Driglasky et on recueille le surnageant. Les boîtes ensemencées sont incubées à 37° C pendant 20 heures.

Les résultats obtenus sont décrits dans les tableaux suivants. L'activité des composés "in vitro" y est

comparée à celle de la norfloxacine. Les concentrations sont données en µg/ml.

TABLEAU II

| MICROORGANISM | Norfloxacine | EXEMPLE 1 | EXEMPLE 3 |
|---|---|---|---|
| Bacillus subtilis ATCC 6633 | 0,25 | ≤0,03 | ≤0.03 |
| Bacillus cereus ATCC 11778 | 1,0 | 0,06 | 0.12 |
| Strep. faecalis ATCC 10541 | 1,0 | 0,12 | 0.50 |
| Staph. aureus ATCC 25178 | 2,0 | 0,06 | 0.06 |
| Staph. epidermidis ATCC 155-1 | 1,0 | ≤0,03 | 0.06 |
| Ps. aeruginosa ATCC 9721 | 0,5 | 0,12 | 1.0 |
| Ps. aeruginosa ATCC 10145 | 1,0 | 0,12 | 0.5 |
| Citr. freundii ATCC 11606 | 0,25 | ≤0,03 | 0,06 |
| Morg. morganii ATCC 8019 | 0,12 | ≤0,03 | 0,12 |
| Proteus vulgaris ATCC 8427 | 0,06 | 0,06 | 0,12 |
| Kleb. pneumoniae ATCC 10031 | 0,03 | ≤0,03 | 0,12 |
| Sal. typhimurium ATCC 14028 | 0,25 | 0,06 | 0,25 |
| Sal. typhi ATCC 6539 | 0,06 | ≤0,03 | 0,06 |
| Escherichia coli ATCC 10799 | 0,25 | ≤0,03 | 0,12 |
| Escherichia coli ATCC 23559 | 0,06 | ≤0,03 | ≤0,03 |
| Ent. aerogenes ATCC 15038 | 0,25 | ≤0,03 | 0,12 |
| Ent. cloacae ATCC 23355 | 0,06 | ≤0,03 | 0,12 |
| Serr. marcescens ATCC 13880 | 0,50 | ≤0,03 | 0,50 |
| Shigella flexnerii ATCC 12022 | 0,12 | ≤0,03 | ≤0,03 |

TABLE II (cont.)

| MICROORGANISM | EXAMPLES | | | | |
|---|---|---|---|---|---|
| | 2 | 5 | 9 | 10 | 22 |
| Bacillus subtilis ATCC 6633 | 0,03 | ≤0,03 | ≤0,03 | 0,03 | 0,03 |
| Bacillus cereus ATCC 11778 | 0,12 | ≤0,03 | ≤0,03 | 0,06 | 0,25 |
| Strep. faecalis ATCC 10541 | 0,25 | 0,25 | 0,12 | 0.06 | 0.50 |
| Staph. aureus ATCC 25178 | 0,12 | 0,06 | 0,06 | 0,06 | 0,06 |
| Staph. epidermidis ATCC 155-1 | 0,12 | 0,06 | ≤0,03 | 0,06 | 0,06 |
| Ps. aeruginosa ATCC 9721 | 0,25 | 0,25 | 0,12 | 0,50 | 0,50 |
| Ps. aeruginosa ATCC 10145 | 0,50 | 0,25 | 0,12 | 0,50 | 0,50 |
| Citr. freundii ATCC 11606 | 0,06 | ≤0,03 | ≤0,03 | 0,06 | 0,12 |
| Morg. morganii ATCC 8019 | 0,06 | ≤0,03 | ≤0,03 | 0,06 | 0,06 |
| Proteus vulgaris ATCC 8427 | 0,12 | 0,06 | 0,06 | 0,12 | 0,12 |
| Kleb. pneumoniae ATCC 10031 | 0,12 | ≤0,03 | ≤0,03 | 0,03 | 0,03 |
| Sal. typhimurium ATCC 14028 | 0,12 | ≤0,03 | ≤0,03 | 0,25 | 0,06 |
| Sal. typhi ATCC 6539 | 0,12 | ≤0,03 | ≤0,03 | 0,25 | 0,03 |
| Escherichia coli ATCC 10799 | 0,06 | ≤0,03 | ≤0,03 | 0,06 | 0,06 |
| Escherichia coli ATCC 23559 | 0,03 | ≤0,03 | ≤0,03 | 0,03 | 0,03 |
| Ent. aerogenes ATCC 15038 | 0,06 | ≤0,03 | ≤0,03 | 0,06 | 0,03 |
| Ent. cloacae ATCC 23355 | 0,12 | ≤0,03 | ≤0,03 | 0,06 | 0,03 |
| Serr. marcescens ATCC 13880 | 0,12 | 0,12 | 0,06 | 0,25 | 0,12 |
| Shigella flexnerii ATCC 12022 | 0,06 | ≤0,03 | ≤0,03 | 0,03 | 0,03 |

TABLE II (cont.)

| MICROORGANISM | EXAMPLES 29 | EXAMPLES 33 |
|---|---|---|
| Bacillus subtilis ATCC 6633 | 0,06 | 0,12 |
| Bacillus cereus ATCC 11778 | 0,06 | 0,25 |
| Strep. faecalis ATCC 10541 | 0,25 | 0,50 |
| Staph. aureus ATCC 25178 | 0,12 | 0,25 |
| Staph. epidermidis ATCC 155-1 | 0,12 | 0,25 |
| Ps. aeruginosa ATCC 9721 | 1,0 | 1,0 |
| Ps. aeruginosa ATCC 10145 | 1,0 | 2,0 |
| Citr. freundii ATCC 11606 | 0,06 | 0,12 |
| Morg. morganii ATCC 8019 | 0,06 | 0,12 |
| Proteus vulgaris ATCC 8427 | 0,12 | 0,25 |
| Kleb. pneumoniae ATCC 10031 | 0,03 | 0,12 |
| Sal. typhimurium ATCC 14028 | 0,06 | 0,25 |
| Sal. typhi ATCC 6539 | 0,03 | 0,12 |
| Escherichia coli ATCC 10799 | 0,12 | 0,25 |
| Escherichia coli ATCC 23559 | 0,06 | 0,12 |
| Ent. aerogenes ATCC 15038 | 0,12 | 0,25 |
| Ent. cloacae ATCC 23355 | 0,03 | 0,12 |
| Serr. marcescens ATCC 13880 | 0,25 | 0,25 |
| Shigella flexnerii ATCC 12022 | 0,03 | 0,12 |

En thérapeutique humaine, la dose d'administration est bien sûr fonction de la susceptibilité de la souche infective, de la nature du composé administré et de la voie d'administration. Elle sera généralement comprise entre environ 0,200 et environ 300 mg pour chaque kilogramme de poids et par jour. Les dérivés de l'invention seront, par exemple, administrés sous forme de comprimés, de solutions ou suspensions, ou bien de gélules.

On indiquera ci-après, à titre d'exemples, deux formes galéniques particulières des dérivés objet de la présente invention.

| Exemple de formule par comprimé: | |
|---|---|
| Composé de l'exemple 5 | 50 mg |
| Cellulose microcristalline | 100 mg |
| Povidone | 15 mg |
| Amidon de blé | 73 mg |
| Dioxyde de silice colloïdale | 50 mg |
| Stéarate de magnésium | 3 mg |
| Poids comprimé | 300 mg |

| Exemple de formule par gélule | |
|---|---|
| Composé de l'exemple 5 | 100 mg |
| Glycéride polyoxyéthylenée | 185 mg |
| Behenate de glycerine | 15 mg |
| Excipient: gélatine molle q.s. | 300 mg |

**Revendications**

1.- Les composés répondant à la formule générale I :

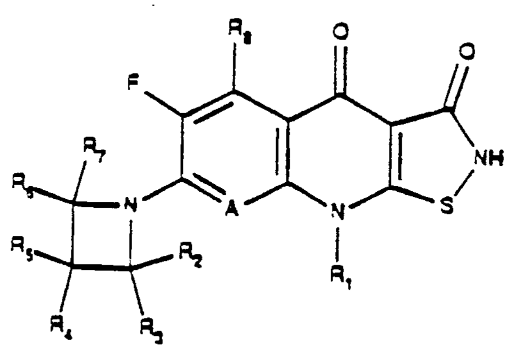

I

qu'on peut écrire dans sa formule tautomérique II,

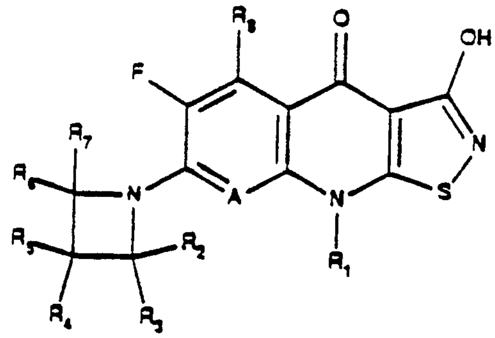

II

dans lesquelles A représente un atome d'azote ou bien un atome de carbone avec un atome d'hydrogène attaché (C-H), ou bien un atome de carbone avec un halogène attaché (C-X), dans ce cas X représente un atome de brome, chlore ou de fluor

$R_1$ représente un radical alkyle ou cycloalkyle inférieur, un radical halogénoalkyle inferieur, un radical aryle ou un radical aryle substitué, notamment par un ou plusieurs atome(s) de fluor,

$R_2$, $R_7$, égaux ou différents, représentent un atome d'hydrogène, ou un radical alkyle inférieur,

$R_3$, $R_5$ et $R_6$ égaux ou différents représentent un atome d'hydrogène, un radical alkyle inférieur, un radical aminoalkyle, un radical alkylamino, un radical alkylaminoalkyle,

$R_4$ représente un atome d'hydrogène, un radical alkyle inférieur, un radical hydroxyle, un radical amino, un radical aminoalkyle, un radical alkylamino, un radical dialkylamino, un radical hétérocyclique azoté pouvant être un cycle de trois à six maillons, un radical alkylaminoalkyle, un radical alkylcarboxamido, et dans ce dernier cas, le radical alkyl pouvant être substitué par un ou plusieurs halogènes, un radical arylsulfonyloxy, un radical alkylsulfonyloxy, un radical carboxamido, pouvant être substitué ou non sur l'azote, ou un radical cyano.

$R_8$, représente un atome d'hydrogène, un radical nitro, un radical amino ou amino substitué ,

A et $R_1$, peuvent former ensemble une liaison réprésentée par un groupe $C-CH_2-CH_2-CHR_9-$ ou $C-O-CH_2-CHR_9-$ dans lesquels $R_9$ représente un atome d'hydrogène ou un radical al kyle inférieur, et dans ce dernier cas on a un centre chiral avec une configuration "R" ou "S",

ainsi que leurs sels d'acides minéraux tels les chlorhydrates, ou d'acides organiques tels que les toluènesulfonates ou méthylsulfonates physiologiquement acceptables.

2.- Les composés répondant à la formule générale I selon la revendication 1 dans laquelle $R_1$ représente cyclopropyle; A représente un atome d'azote ou bien un atome de carbone avec un atome d'hydrogène attaché (C-H), ou bien un atome de carbone avec un atome de chlore (C-Cl) ou de fluor (C-F) attaché; $R_2$, $R_7$ et $R_8$ représentent un atome d'hydrogène; $R_3$, $R_5$ et $R_6$ égaux ou différents sont sélection parmi un atome d'hydrogène ou un radical alkyle inférieur; et $R_4$ sélectionné parmi un atome d'hydrogène, un radical hydroxyle, un radical amino, un radical aminoalkyle, un radical alkylamino, un radical dialkylamino ou un radical alkylaminoalkyle.

3.- Les composés répondant à la formule générale I selon la revendication 1 dans laquelle A et $R_1$ forment ensemble une liaison réprésentée par un groupe $-C-O-CH_2-CH(CH_3)-$; $R_2$, $R_7$ et $R_8$ représentent un atome d'hydrogène; $R_3$, $R_5$ et $R_6$ égaux ou différents sont sélectionné parmi un atome d'hydrogène ou un radical alkyle inférieur; et $R_4$ sélectionné parmi un atome d'hydrogène, un radical hydroxyle, un radical amino, un radical aminoalkyle, un radical alkylamino, un radical dialkylamino ou un radical alkylaminoalkyle.

4.- Les composés répondant à la formule générale I selon les revendications 1 à 3, séléctionnés parmi le groupe suivant:

- 9-cyclopropyl-6-fluoro-7-(3-amino-1-azétidinyl)-2,3,4,9-tétrahydroisothiazolo [5,4-b] quinoline-3,4-dione
- 9-cyclopropyl-6-fluoro-7-(3-méthylamino-1-azétidinyl)-2,3,4,9-tétrahydroisothiazolo [5,4-b] quinoline-3,4-dione,
- 9-cyclopropyl-6-fluoro-7-(3-diméthylamino-1-azétidinyl)-2,3,4,9-tétrahydroisothiazolo [5,4-b] quinoline-3,4-dione
- 9-cyclopropyl-6-fluoro-7-(trans-3-amino-2-méthyl-1-azétidinyl)-2,3,4,9-tétrahydroisothiazolo [5,4b] quinoline-3,4-dione
- 9-cyclopropyl-6-fluoro-7-(3-amino-3-méthyl-1-azétidinyl)-2,3,4,9-tétrahydroisothiazolo [5,4-b] quinoline-3,4-dione
- 9-cyclopropyl-6-fluoro-7-(3-méthylamino-3-méthyl-1-azétidinyl)-2,3,4,9-tétrahydroisothiazolo [5,4-b] quinoline-3,4-dione.
- 9-cyclopropyl-6-fluoro-7-(3-ethylamino-1-azétidinyl)-2,3,4,9-tétrahydroisothiazolo [5,4-b] quinoline-3,4-dio-

ne.

■ 9-cyclopropyl-6,8-difluoro-7-(trans-3-amino-2-méthyl-1-azétidinyl)-2,3,4,9-tétrahydroisothiazolo [5,4-b] quinoline-3,4-dione.

■ 9-cyclopropyl-6,8-difluoro-7-(3-amino-3-méthyl-1-azétidinyl)-2,3,4,9-tétrahydroisothiazolo [5,4-b] quinoline-3,4-dione.

5.- Procédé de préparation des composés, selon l'une quelconque des revendications 1 à 4, caractérisé en ce qu'il comprend la réaction d'un composé de formule générale III

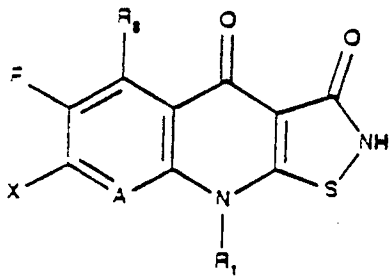

III

dans laquelle A, $R_1$ et $R_8$ ont lest significations mentionnées dans la revendication 1 et X représente un atome d'halogène, de preférence un chlore ou un fluor, avec une azétidine, de formule générale IV

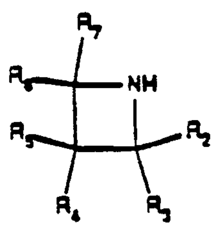

IV

dans laquelle $R_2$, $R_3$, $R_4$, $R_5$, $R_6$ et $R_7$ ont les significations mentionées dans la revendication 1.

6. Procédé selon la revendication 5, caractérisé en ce que la réaction s'effectue en présence d'un solvant organique choisi de préférence parmi le diméthylsulfoxyde, la diméthylformamide, la pyridine, les trialkylamines, comme la triéthylamine, le chlorure de méthylène, le chloroforme, l'acétonitrile ou bien des éthers comme le tétrahydrofuranne, le dioxanne, ou des mélanges de ces solvants.

7. Procédé selon la revendication 5 ou 6, caractérisé en ce que les températures réactionnelles varient entre la température ambiante et la température de reflux du solvant, et le temps réactionnel est compris entre 1 heure et 24 heures.

8.- A titre de médicaments, les dérivés de formule générale I et leurs sels thérapeutiquement acceptables, selon les revendications 1 à 4, en particulier à titre de médicaments destinés au traitement de certaines maladies infectieuses.

9.- Compositions pharmaceutiques, caracterisées par le fait qu'elles contiennent, outre un support pharmaceutiquement acceptable, au moins un dérivé de formule générale I ou l'un de ses sels physiologiquement acceptables, selon l'une des revendications 1 à 4.

10.- Utilisation des dérivés de formule générale I et leurs sels physiologiquement acceptables, selon l'une des revendications 1 à 4, pour la fabrication de médicaments destinés au traitement des infections microbiennes.

Office européen des brevets

# RAPPORT DE RECHERCHE EUROPEENNE

Numero de la demande

EP 90 40 1036

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 227 088 (ABBOTT)<br>* Revendications 1,8 *<br>--- | 1,9 | C 07 D 513/04<br>C 07 D 513/14<br>C 07 D 513/16<br>A 61 K 31/435 //<br>(C 07 D 513/04<br>C 07 D 275:00<br>C 07 D 221:00 )<br>(C 07 D 513/14<br>C 07 D 275:00<br>C 07 D 221:00<br>C 07 D 221:00 )<br>(C 07 D 513/16<br>C 07 D 275:00<br>C 07 D 221:00<br>C 07 D 221:00 )<br>(C 07 D 513/16<br>C 07 D 275:00<br>-/- |
| D,A | EP-A-0 228 661 (ABBOTT)<br>* Revendications 1,7 *<br>----- | 1,9 | |
| | | | **DOMAINES TECHNIQUES RECHERCHES (Int. Cl.5)** |
| | | | C 07 D 513/00<br>A 61 K 31/00 |

Le présent rapport a été établi pour toutes les revendications

| Lieu de la recherche | Date d'achèvement de la recherche | Examinateur |
|---|---|---|
| LA HAYE | 12-07-1990 | ALFARO I. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 90 40 1036

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| | | | C 07 D 265:00<br>C 07 D 221:00 ) |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 12-07-1990 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant